# EUROPEAN PATENT APPLICATION

(11) **EP 2 168 680 A1**
(43) Date of publication of application: **31.03.2010**
(21) Application number: 08790988.3
(22) Date of filing: 09.07.2008
(51) Int. Cl.: B01J 31/22, B01J 31/24, B01J 31/30, C07C 1/30, C07C 1/32, C07C 15/14, C07B 61/00

(54) **CATALYST COMPOSITION AND METHOD FOR PRODUCING CROSS-COUPLING COMPOUND USING THE SAME**

(30) Priority: 11.07.2007 JP 2007181653
(71) Applicant: Kyoto University, Kyoto-shi, Kyoto 606-8501 (JP); Tosoh Corporation, Yamaguchi 746-8501 (JP); Tosoh Finechem Corporation, Shunan-shi Yamaguchi 746-0006 (JP)
(72) Inventor: NAKAMURA, Masaharu, Uji-shi Kyoto 611-0011 (JP); HATAKEYAMA, Takuji, Uji-shi Kyoto 611-0011 (JP); EGUCHI, Hisao, Shunan-shi Yamaguchi 746-0006 (JP); YANO, Hirokazu, Shunan-shi Yamaguchi 746-8501 (JP)
(74) Representative: Browne, Robin Forsythe
(86) International application number: PCT/JP2008/062401
(87) International publication number: WO 2009/008447

(57) **Abstract**

A catalyst composition for a cross-coupling reaction comprising a nickel metal source, a fluorine source, and a nitrogen-containing heterocyclic compound having a specific structure, or a phosphine compound. Using this catalyst composition, a cross-coupled compound R⁵-R⁶ (wherein R⁵ and R⁶ are an aryl or heteroaryl group, or a linear, branched or cyclic alkyl group or a linear, branched or cyclic alkenyl group) is produced with a high selectivity by allowing an organometallic compound of the formula (2) R⁵-MY¹ or the formula (3) R⁵-M-R⁵ (wherein R⁵ is the same as defined above, M is a Mg or Zn atom, and Y¹ is a halogen atom) to react with a compound of the formula (4) R⁶-Y² (wherein R⁶ is the same as defined above, and Y² is a halogen atom, a methanesulfonate group, a toluenesulfonate group or a trifluoromethanesulfonate group).

## Description

### Technical Field

This invention relates to a catalyst composition exhibiting a high catalytic activity and selectivity for a cross-coupling reaction in organic syntheses, and a process for producing a coupled compound using the catalyst composition.
Various functional compounds can be produced with an enhanced efficiency by the process according to the present invention. More specifically, unsymmetrical biaryl compounds and other compounds which are useful, for example, as liquid crystal or a raw material for medicines can be produced with an enhanced efficiency.

### Background Art

Unsymmetrical biaryl compounds characterized as having stability due to their aromatic rings and fixed molecular structure are very useful as electronic materials, and intermediates for the synthesis of medicines and pesticides, and various functional compounds.
Unsymmetrical biaryl compounds have heretofore been produced widely by a process comprising cross-coupling an organic halide with an organometallic compound in the presence of a catalyst. Among others, processes utilizing a coupling reaction of an organometallic compound such as an organomagnesium compound or a organozinc compound with an organic halide are well known as an inexpensive cross-coupling reaction process (see, for example, JP H04-173756 A1, especially examples, and JP 2000-95713 A1, especially examples, and Bulletin of the Chemical Society of Japan, (Japan), 1976, vol. 49, p1958-1969, especially Tables 6 and 7).

In the above-mentioned processes, a nickel catalyst or a palladium catalyst are usually used as a catalyst. However, these processes have problems. That is, in the case when a nickel catalyst is used, homo-coupled compounds derived from an organometallic compound or an organic halide are produced in large amounts by side reactions. Purification of the thus-synthesized unsymmetrical biaryl compounds required for use as liquid crystal and intermediate for medicines is very costly. In the case when a palladium catalyst is used, the catalyst is expensive and the process is costly and industrially not advantageous.

Recently, as for the cross-coupling reaction between an organometallic compound with an organic halide, a process using a catalyst comprising a combination of a nickel compound with an N-heterocyclic compound has been proposed (see, for example, Angewandte Chemie International Edition, Germany, 2000, vol. 39, p1602-1604, especially, supporting information Tables 1 to 4). The cross-coupling reaction in this proposed process gives a relatively high yield of the target compound, but the above-mentioned problem of production of undesirable homo-coupled compounds by side reactions still remains unsolved. The experiments conducted by the present inventors revealed that a large amount of the homo-coupled compounds are produced by side reactions. Therefore, the proposed process is still industrially not advantageous.

### Disclosure of the Invention

### Problems to Be Solved by the Invention

An object of the present invention is to provide a catalyst composition exhibiting high activity and selectivity for the cross-coupling reaction for which an industrially satisfying process has not been heretofore proposed, especially a catalyst which does not produce homo-coupled compounds by side reactions from an organometallic compound or an organic halide.
Another object of the present invention is to provide a process for producing a cross-coupled compound using the above-mentioned catalyst composition.

### Means for Solving the Problems

The present inventors made extensive research to solve the foregoing problems of the prior art, and have found that a novel catalyst composition comprising a nickel metal source, a fluorine source and a nitrogen-containing heterocyclic compound having a specific structure, or a phosphine compound exhibits very high activity and selectivity for the cross-coupling reaction of an organometallic compound such as an organomagnesium compound or a organozinc compound with an organic halide, and said catalyst does not cause the problem of producing homo-coupled compounds by side reactions.

In accordance with the present invention, there is provided a catalyst composition for a cross-coupling reaction comprising a nickel metal source, a fluorine source, and a nitrogen-containing heterocyclic compound represented by the following formula (1A) or (1B), or a phosphine compound:

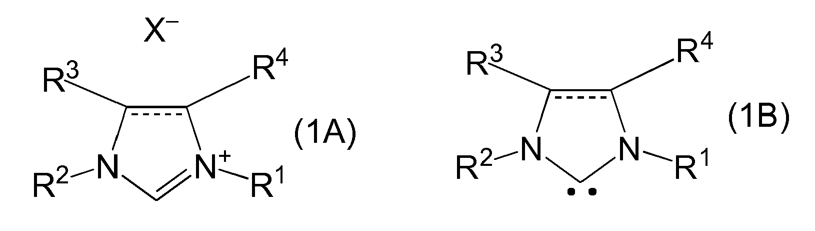

wherein,

R¹ and R² may be the same or different and represent a substituted or unsubstituted aryl group, a substituted or unsubstituted heteroaryl group, a substituted or unsubstituted linear, branched or cyclic alkyl group, or a substituted or unsubstituted linear, branched or cyclic alkenyl group,
R³ and R⁴ may be the same or different and represent a hydrogen atom, a substituted or unsubstituted aryl group, a substituted or unsubstituted heteroaryl group, a substituted or unsubstituted linear, branched or cyclic alkyl group, a substituted or unsubstituted linear, branched or cyclic alkenyl group, a substituted or unsubstituted alkoxy group, a substituted or unsubstituted aryloxy group, or a substituted or unsubstituted silyl group, and R³ and R⁴ may be bonded together to form a ring with the carbon atoms to which R³ and R⁴ are bonded, and

-̅ -̅ -̅ -̅ -̅ -̅

represents a single bond or a double bond, and
X⁻ represents a univalent anion.

In accordance with the present invention, there is further provided a process for producing a cross-coupled compound represented by the following general formula (5):

R⁵-R⁶ (5)

wherein R⁵ and R⁶ represent a substituted or unsubstituted aryl group, a substituted or unsubstituted heteroaryl group, a substituted or unsubstituted linear, branched or cyclic alkyl group, or a substituted or unsubstituted linear, branched or cyclic alkenyl group,

which comprises allowing an organometallic compound represented by the following general formula (2) or (3):

R⁵ - MY¹ (2)

R⁵ - M - R⁵ (3)

wherein R⁵ is the same as defined above, M represents a magnesium metal or a zinc metal, and Y¹ represents a halogen atom,

to react with a compound represented by the following general formula (4):

R⁶-Y² (4)

wherein R⁶ is the same as defined above, and Y² represents a halogen atom, a methanesulfonate group, a toluenesulfonate group or a trifluoromethanesulfonate group,
in the presence of at least one catalyst composition as mentioned above.

### Effect of the Invention

The catalyst composition according to the present invention is a novel catalyst composition, which has not heretofore been proposed, and exhibits high activity and selectivity for a cross-coupling reaction. That is, this catalyst composition suppresses to a great extent the production of undesirable homo-coupled compounds by side reactions, to give the desired cross-coupled compound with a very high selectivity.
By using the catalyst composition according to the present invention, the cross-coupling reaction, for which an industrially satisfying process has not heretofore been proposed, can be industrially advantageously conducted. Especially, unsymmetrical biaryl compounds and other compounds which are useful as liquid crystal or a raw material for medicines can be produced with enhanced yield and selectivity. The catalyst composition according to the present invention does not contain an expensive metal such as palladium, therefore the coupling reaction using the catalyst composition is advantageous from an economical and industrial viewpoint.

### Best Mode for Carrying Out the Invention

The invention will be described in detail.
[Catalyst Composition for Cross-coupling Reaction]
The catalyst composition of the present invention is classified into two types.
A first type of the catalyst composition comprises a nickel metal source, a fluorine source and a nitrogen-containing heterocyclic compound of the following general formula (1A) or (1B):

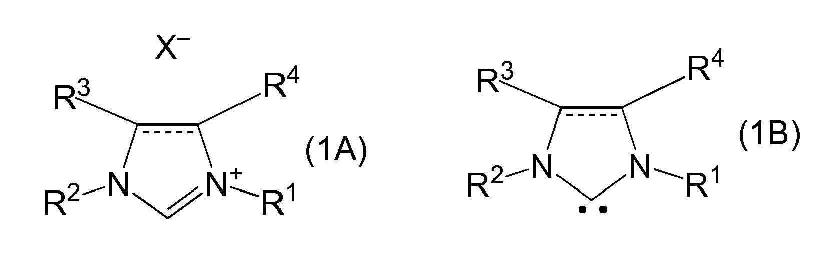

In the above formulae, R¹ and R² may be the same or different and represent a substituted or unsubstituted aryl group, a substituted or unsubstituted heteroaryl group, a substituted or unsubstituted linear, branched or cyclic alkyl group, or a substituted or unsubstituted linear, branched or cyclic alkenyl group. R³ and R⁴ may be the same or different and represent a hydrogen atom, a substituted or unsubstituted aryl group, a substituted or unsubstituted heteroaryl group, a substituted or unsubstituted linear, branched or cyclic alkyl group, a substituted or unsubstituted linear, branched or cyclic alkenyl group, a substituted or unsubstituted alkoxy group, a substituted or unsubstituted aryloxy group, or a substituted or unsubstituted silyl group. R³ and R⁴ may be bonded together to form a ring with the carbon atoms to which R³ and R⁴ are bonded.

-̅ -̅ -̅ -̅ -̅ -̅

represents a single bond or a double bond, and X⁻ represents a univalent anion.
The above-mentioned nitrogen-containing heterocyclic compound represented by the formula (1A) or (1B) is hereinafter referred to as "nitrogen-containing heterocyclic compound" when appropriate, and the first type catalyst composition containing this nitrogen-containing heterocyclic compound according to the present invention is hereinafter referred to as "first catalyst composition" when appropriate.

The nickel metal source, which is one essential ingredient of the first catalyst composition, is capable of giving a nickel metal to a catalyst system. The nickel source is not particularly limited, and is usually selected from nickel salts with a valency of 0 to 2, and complex compounds derived from the nickel salts.
As specific examples of the nickel metal source, there can be mentioned nickel(0) powder; nickel halides such as nickel(II) fluoride, nickel(II) chloride, nickel(II) bromide and nickel(II) iodide; inorganic nickel salts such as nickel(II) sulfate, nickel (II) nitrate, nickel (II) perchlorate and nickel (II) sulfide; and organic acid nickel salts such as nickel(II) formate, nickel(II) oxalate, nickel(II) acetate, nickel(II) fumarate, nickel(II) lactate, nickel(II) gluconate, nickel(II) benzoate, nickel (II) stearate, nickel (II) sulfamate, nickel (II) amidosulfate, nickel(II) carbonate and nickel(II) acetylacetonate.

The complex compounds derived from the nickel salts are not particularly limited, but usually include those which are represented by the following general formula (7):

**LcNiXd** (7)

wherein L represents a phosphine ligand, an amine ligand or a carbonyl ligand, and, when L is plural, they may be the same or different. These ligands may be crosslinked. c represents an integer in the range of 0 to 6. Ni has a valency of 0 to 2. X represents a halogen atom, a hydrogen atom or a hydrocarbon group having 1 to 20 carbon atoms, and, when X is plural, they may be the same or different. d represents a number equal to the number for valency of Ni.

As specific examples of the Ni complex compound, there can be mentioned bis(1,5-cyclooctadiene)nickel(0), bis(allyl)nickel(0), tetracarbonylnickel(0), tricarbonyl(triphenylphosphine)nickel(0), dicarbonylbis(triphenylphosphine)nickel(0), tetrakis(triphenylphosphine)nickel(0), bis[bis(tricyclohexylphosphine)nickel(0) dinitride, (ethylene)bis(triphenylphosphine)nickel(0), (cyclododecatriene)nickel(0), tris(triphenylphosphine)nickel(I) chloride, tris(triphenylphosphine)nickel(I) bromide, tris(triphenylphosphine)nickel(I) iodide, bis(triphenylphosphine)nickel(II) dichloride, bis(triphenylphosphine)nickel(II) dibromide, bis(triphenylphosphine)nickel(II) diiodide, [1,2-bis(diphenylphosphino)ethane]nickel(II) dichloride, [1,2-bis(diphenylphosphino)ethane]nickel(II) dibromide, [1,2-bis(diphenylphosphino)ethane]nickel(II) diiodide, [1,3-bis(diphenylphosphino)propane]nickel(II) dichloride, [1,3-bis(diphenylphosphino)propane]nickel(II) dibromide, [1,3-bis(diphenylphosphino)propane]nickel(II) diiodide, bis(tri-t-butylphosphine)nickel(II) dichloride, bis(tri-t-butylphosphine)nickel(II) dibromide, bis(tri-t-butylphosphine)nickel(II) diiodide, [1,2-bis(di-t-butylphosphino)ethane]nickel(II) dichloride, [1,2-bis(di-t-butylphosphino)ethane]nickel(II) dibromide, [1,2-bis(di-t-butylphosphino)ethane]nickel(II) diiodide, [1,3-bis(di-t-butylphosphino)propane]nickel(II) dichloride, [1,3-bis(di-t-butylphosphino)propane]nickel(II) dibromide, [1,3-bis(di-t-butylphosphino)propane]nickel(II) diiodide, [1,2-bis(dicyclohexylphosphino)ethane]nickel(II) dichloride, [1,2-bis(dicyclohexylphosphino)ethane]nickel(II) dibromide, [1,2-bis(dicyclohexylphosphino)ethane]nickel(II) diiodide, [1,3-bis(dicyclohexylphosphino)propane]nickel(II) dichloride, [1,3-bis(dicyclohexylphosphino)propane]nickel(II) dibromide, [1,3-bis(dicyclohexylphosphino)propane]nickel(II) diiodide, bis(tricyclohexylphosphine)nickel(II) dichloride, bis(tricyclohexylphosphine)nickel(II) dibromide, bis(tricyclohexylphosphine)nickel(II) diiodide, bis(trimethylphosphine)nickel(II) dichloride, bis(trimethylphosphine)nickel(II) dibromide, bis(trimethylphosphine)nickel(II) diiodide, bis(triethylphosphine)nickel(II) dichloride, bis(triethylphosphine)nickel(II) dibromide, bis(triethylphosphine)nickel(II) diiodide, bis(tri-i-propylphosphine)nickel(II) dichloride, bis(tri-i-propylphosphine)nickel(II) dibromide, bis(tri-i-propylphosphine)nickel(II) diiodide, bis(tri-n-butylphosphine)nickel(II) dichloride, bis(tri-n-butylphosphine)nickel(II) dibromide, bis(tri-n-butylphosphine)nickel(II) diiodide, bis(methyldiphenylphosphine)nickel(II) dichloride, bis(methyldiphenylphosphine)nickel(II) dibromide, bis(methyldiphenylphosphine)nickel(II) diiodide, bis(dimethylphenylphosphine)nickel(II) dichloride, bis(dimethylphenylphosphine)nickel(II) dibromide, bis(dimethylphenylphosphine)nickel(II) diiodide, (phenyl)bis(triphenylphosphine)nickel(II) chloride, (phenyl)bis(triphenylphosphine)nickel(II) bromide, (phenyl)bis(triphenylphosphine)nickel(II) iodide, dimethylbis(trimethylphosphine)nickel(II), tetramethylenebis(triphenylphosphine)nickel(II), (cyclopentadienyl)(triphenylphosphine)nickel(II) chloride, methyl(cyclopentadienyl)(triphenylphosphine)nickel(II), bis(tricyclohexylphosphine)nickel(II) chloride hydride, (2,2'-bipyridyl)nickel(II) dichloride, (2,2'-bipyridyl)nickel(II) dibromide, (2,2'-bipyridyl)nickel(II) diiodide, diethyl(2,2'-bipyridyl)nickel(II), bis(triethylamine)nickel dichloride, bis(triethylamine)nickel dibromide, bis(triethylamine)nickel diiodide, bis(pyridine)nickel dichloride, bis(pyridine)nickel dibromide, bis(pyridine)nickel diiodide, (N,N,N',N'-tetramethylethylenediamine)nickel dichloride, (N,N,N',N'-tetramethylethylenediamine)nickel dibromide, and (N,N,N',N'-tetramethylethylenediamine)nickel diiodide.
The above-listed complex compounds may be used either alone or as a mixture thereof.

The fluorine source, which is one essential ingredient of the first catalyst composition, is capable of giving a fluorine atom to a catalyst system.
The fluorine source is not particularly limited, and, as specific examples thereof, there can be mentioned fluorine gas, hydrofluoric acid, lithium fluoride, sodium fluoride, potassium fluoride, rubidium fluoride, cesium fluoride, beryllium fluoride, magnesium fluoride, calcium fluoride, strontium fluoride, barium fluoride, scandium fluoride, yttrium fluoride, lanthanum fluoride, titanium fluoride, zirconium fluoride, hafnium fluoride, vanadium fluoride, niobium fluoride, tantalum fluoride, chromium fluoride, molybdenum fluoride, tungsten fluoride, manganese fluoride, ruthenium fluoride, osmium fluoride, rhodium fluoride, iridium fluoride, nickel fluoride, palladium fluoride, copper fluoride, silver fluoride, zinc fluoride, cadmium fluoride, boron fluoride, aluminum fluoride, gallium fluoride, indium fluoride, thallium fluoride, tin fluoride, lead fluoride, antimony fluoride, ammonium fluoride, potassium titanium fluoride, samarium fluoride, hydrogen fluoride-pyridine, hydrosilicofluoric acid, sodium silicofluoride, potassium silicofluoride, ammonium silicofluoride, magnesium silicofluoride, hydroborofluoric acid, sodim borofluoride, potassium borofluoride, iron borofluoride, zinc borofluoride, copper borofluoride, tin borofluoride, ammonium borofluoride and lithium hexafluorophosphate.
The above-listed compounds may be used either alone or as a mixture thereof.

Nickel fluoride is capable of giving a nickel metal and simultaneously a fluorine atom to the catalyst system, therefore, nickel fluoride can be used alone as the nickel metal source and the fluorine source. Nickel fluoride is especially preferable in view of low cost, high reactivity and high stability.
The first catalyst composition comprises, in addition to the above-mentioned nickel metal source and fluorine source, a nitrogen-containing heterocyclic compound of the following general formula (1A) or (1B) as an essential ingredient.

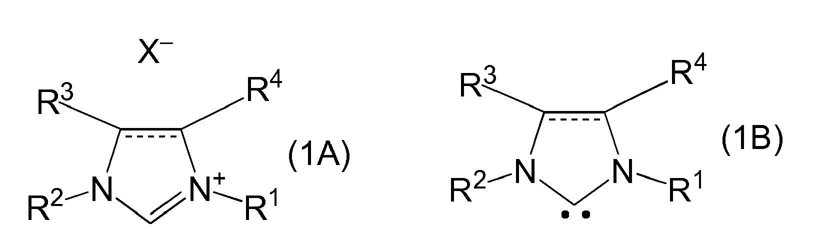

In the above formulae (1A) and (1B), a substituted or unsubstituted aryl group for R¹, R², R³ and R⁴ is not particularly limited, and includes, for example, substituted or unsubstituted aryl groups having 1 to 4 rings and 6 to 18 carbon atoms. As specific examples of the aryl groups, there can be mentioned phenyl, naphthyl, anthracenyl, azulenyl, pyrenyl, phenanthrenyl and fluorenyl groups.
Optional number of hydrogen atoms in these aryl groups may be substituted by substituents which include, for example, a halogen atom, a substituted or unsubstituted liner, branched or cyclic alkyl group, a substituted or unsubstituted aryl group, a substituted or unsubstituted heteroaryl group, a substituted or unsubstituted liner, branched or cyclic alkenyl group, a hydroxyl group, an alkoxy group, an amino group, a cyano group, a carbonyl group, a carboxyl group, an ester group and an silyl group.

A substituted or unsubstituted heteroaryl group for R¹, R², R³ and R⁴ is not particularly limited, and includes, for example, substituted or unsubstituted heteroaryl groups having 1 to 4 rings and 6 to 18 carbon atoms. As specific examples of the heteroaryl groups, there can be mentioned pyridyl, pyrimidyl, pyridazyl, pyrazyl, triazyl, benzofuranyl, indolyl, benzothiofenyl, quinolyl, isoquinolyl, carbazolyl, acridinyl, phenanthrolinyl and phenothiazinyl groups.
Optional number of hydrogen atoms in these heteroaryl groups may be substituted by substituents which include, for example, a halogen atom, a substituted or unsubstituted liner, branched or cyclic alkyl group, a substituted or unsubstituted aryl group, a substituted or unsubstituted heteroaryl group, a substituted or unsubstituted liner, branched or cyclic alkenyl group, a hydroxyl group, an alkoxy group, an amino group, a cyano group, a carbonyl group, a carboxyl group and an ester group.

A substituted or unsubstituted linear, branched or cyclic alkyl group for R¹, R², R³ and R⁴ is not particularly limited, and includes, for example, linear, branched or cyclic alkyl groups having 1 to 20 carbon atoms. As specific examples of the alkyl groups, there can be mentioned methyl, ethyl, propyl, isopropyl, butyl, isobutyl, t-butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, eicosyl, cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentadecyl, cyclohexadecyl, cycloheptadecyl, cyclooctadecyl, cyclononadecyl and cycloeicosyl groups. Further, the substituted or unsubstituted alkyl group includes polycyclic hydrocarbon group such as a norbornyl group and a adamantyl group.
Optional number of hydrogen atoms in these alkyl groups may be substituted by substituents which include, for example, a halogen atom, a substituted or unsubstituted liner, branched or cyclic alkyl group, a substituted or unsubstituted aryl group, a substituted or unsubstituted heteroaryl group, a substituted or unsubstituted liner, branched or cyclic alkenyl group, a hydroxyl group, an alkoxy group, an amino group, a cyano group, a carbonyl group, a carboxyl group and an ester group.

A substituted or unsubstituted liner, branched or cyclic alkenyl group for R¹, R², R³ and R⁴ is not particularly limited, and includes, for example, alkenyl groups having 2 to 20 carbon atoms. As specific examples of the alkenyl groups, there can be mentioned vinyl, propenyl, butenyl, pentenyl, hexenyl, heptenyl, octenyl, nonenyl, decenyl, undecenyl, dodecenyl, tridecenyl, tetradecenyl, pentadecenyl, hexadecenyl, heptadecenyl, octadecenyl, nonadecenyl, eicosenyl, cyclopropenyl, cyclobutenyl, cyclopentenyl, cyclohexenyl, cycloheptenyl, cyclooctenyl, cyclononenyl, cyclodecenyl, cycloundecenyl, cyclododecenyl, cyclotridecenyl, cyclotetradecenyl, cyclopentadecenyl, cyclohexadecenyl, cycloheptadecenyl, cyclooctadecenyl, cyclononadecenyl and cycloeicosenyl groups. Further, the substituted or unsubstituted alkenyl group includes polycyclic hydrocarbon group such as a norbornenyl group.
Optional number of hydrogen atoms in these alkenyl groups may be substituted by substituents which include, for example, a halogen atom, a substituted or unsubstituted liner, branched or cyclic alkyl group, a substituted or unsubstituted aryl group, a substituted or unsubstituted heteroaryl group, a substituted or unsubstituted liner, branched or cyclic alkenyl group, a hydroxyl group, an alkoxy group, an amino group, a cyano group, a carbonyl group, a carboxyl group and an ester group.

A substituted or unsubstituted alkoxy group for R³ and R⁴ is represented by the following formula (8):

**OR⁷** (8)

wherein R⁷ represents a substituted or unsubstituted linear, branched or cyclic alkyl group. The substituted or unsubstituted linear, branched or cyclic alkyl group includes, for example, those which are recited above.
The substituted or unsubstituted alkoxy group includes, for example, alkoxy groups having 1 to 12 carbon atoms, preferably 1 to 6 carbon atoms, and, as specific examples thereof, there can be mentioned methoxy, ethoxy, isopropoxy, t-butoxy and benzyloxy groups.

A substituted or unsubstituted aryloxy group for R³ and R⁴ is represented by the following formula (9):

**OR⁸** (9)

wherein R⁸ represents a substituted or unsubstituted aryl group. The substituted or unsubstituted aryl group R⁸ includes, for example, substituted or unsubstituted aryl groups having 1 to 4 rings and 6 to 18 carbon atoms, and, as specific examples thereof, those which are recited above as examples of the aryl groups for R³ and R⁴ are mentioned.
The substituted or unsubstituted aryloxy group of the formula (9) includes, for example, a phenoxy group and a 2,4,6-trimethylphenoxy group.

A substituted or unsubstituted silyl group for R³ and R⁴ is not particularly limited, and includes, for example, silyl groups having three substituents selected from the group consisting of alkyl groups and aryl groups. More specifically, such silyl group includes, for example, those which have three substituents selected from the group consisting of alkyl groups having 1 to 6 carbon atoms, preferably 1 to 4 carbon atoms, and aryl groups, examples of which are recited above. The three substituents may be the same or different. As specific examples of the substituted or unsubstituted silyl group, there can be mentioned trimethylsilyl, triethylsilyl, t-butyldimethylsilyl, triphenylsilyl and triisopropylsilyl groups.
R³ and R⁴ may be bonded together to form a ring with the carbon atoms to which R³ and R⁴ are bonded.

As specific examples of the univalent anion represented by X⁻, there can be mentioned F⁻, Cl⁻, Br⁻, I⁻, [OSO₂CH₃]⁻, [OSO₂CF₃]⁻, [OSO₂C₆H₄CH₃]⁻, [N(SO₂CF₃)₂]⁻, [N(SO₂C₆H₄CH₃)₂]⁻, [N(SO₂CH₃)₂]⁻, BF₄⁻, BAr₄⁻, PF₆⁻, AsF₆⁻, SbF₆⁻, C₆F₅ and ClO₄⁻.

As specific examples of the nitrogen-containing heterocyclic compound represented by the formula (1A) or (1B), there can be mentioned 1,3-bis(2,6-diisopropylphenyl)imidazolinium chloride, 1,3-bis(2,6-diisopropylphenyl)imidazolinium tetrafluoroborate, 1,3-bis(2,6-diisopropylphenyl)imidazolidin-2-ylidene, 1,3-bis(2,6-diisopropylphenyl)imidazolium chloride, 1,3-bis(2,6-diisopropylphenyl)imidazolium tetrafluoroborate, 1,3-bis(2,6-diisopropylphenyl)imidazol-2-ylidene, 1,3-bis(2,4,6-trimethylphenyl)imidazolinium chloride, 1,3-bis(2,4,6-trimethylphenyl)imidazolinium tetrafluoroborate, 1,3-bis(2,4,6-trimethylphenyl)imidazolidin-2-ylidene, 1,3-bis(2,4,6-trimethylphenyl)imidazolium chloride, 1,3-bis(2,4,6-trimethylphenyl)imidazolium tetrafluoroborate, 1,3-bis(2,4,6-trimethylphenyl)imidazol-2-ylidene, 1,3-di-t-butylimidazolinium chloride, 1,3-di-t-butylimidazolinium tetrafluoroborate, 1,3-di-t-butylimidazolidin-2-ylidene, 1,3-di-t-butylimidazolium chloride, 1,3-di-t-butylimidazolium tetrafluoroborate, 1,3-di-t-butylimidazol-2-ylidene, 1,3-bis(1-adamantyl)imidazolinium tetrafluoroborate, 1,3-bis(1-adamantyl)imidazolium tetrafluoroborate and 1,3-bis(1-adamantyl)benzimidazolinium tetrafluoroborate.

Of the above-recited nitrogen-containing heterocyclic compounds, especially preferable are those in which R¹ and R² are an aryl group, preferably a phenyl group, having 1 to 3 substituents, preferably alkyl groups having 1 to 6 carbon atoms, or are an alkyl group, preferably an alkyl group having 1 to 6 carbon atoms, and R³ and R⁴ are a hydrogen atom.
The above-recited nitrogen-containing heterocyclic compounds are publicly known compounds or can easily be prepared by known processes.
The relative amounts of the nickel metal source, the fluorine source and the nitrogen-containing heterocyclic compound in the first catalyst composition are appropriately chosen in the following ranges: fluorine atom 1 to 10 moles, preferably 1 to 5 moles, per 1 mole of the nickel metal source, and nitrogen-containing heterocyclic compound 1 to 10 moles, preferably 1 to 5 moles, per 1 mole of the nickel metal source.

The first catalyst composition can be prepared by combining the above-mentioned nickel metal source, fluorine source and nitrogen-containing heterocyclic compound. The procedure of combining the three ingredients includes, for example, a procedure of mixing together the above-mentioned three ingredients, and a procedure of adding the three ingredients separately in a solution for a cross-coupling reaction thereby forming the first catalyst composition within the reactive solution. The state of the three ingredients is not particularly limited, and they may be present discretely, or at least part of the three ingredients may form a complex.

The first catalyst composition may further comprise an organic phosphorus compound. The organic phosphorus compound includes, for example, phosphines and phosphites, and, as specific examples thereof, there can be mentioned phosphines such as triphenylphosphine, trimethylphosphine, tricyclohexylphosphine, tri-t-butylphosphine, bisdiphenylphosphinoethane, and bisdiphenylphosphinopropane; and phosphites such as trimethoxyphosphite, triethoxyphosphite and triphenoxyphosphite.
The relative amounts of the nitrogen-containing heterocyclic compound and the organic phosphorus compound are such that the amount of the nitrogen-containing heterocyclic compound is usually in the range of 1 to 5 moles, preferably 2 to 3 moles, per 1 mole of the organic phosphorus compound.

The first catalyst composition may further comprise, as optional ingredients, an amine compound such as pyridine, triethylamine or N,N,N',N'-tetramethylethylenediamine; a typical metal halide such as zinc chloride, zinc bromide or sodium iodide; and unsaturated hydrocarbons such as ethylene, styrene, butadiene, cyclooctadiene, norbornadiene or diphenylacetylene. In the case when the first catalyst composition comprises the above-mentioned optional ingredients, the amounts of the optional ingredients are appropriately chosen in the range of 1 to 99% by mole based on the total catalyst composition.

The second type catalyst composition according to the present invention comprises a nickel metal source, a fluorine source and a phosphine compound. The second type catalyst composition containing the phosphine compound is hereinafter referred to as "second catalyst composition" when appropriate.
The nickel metal source and the fluorine source in the second catalyst composition are the same as mentioned above for the first catalyst composition.
In the second catalyst composition, nickel fluoride is capable of giving a nickel metal and simultaneously a fluorine atom to the catalyst system, therefore, nickel fluoride can be used alone as the nickel metal source and the fluorine source, as explained for the first catalyst composition. Nickel fluoride is especially preferable in view of low cost, high reactivity and high stability.

The phosphine compound in the second catalyst composition is represented by the following formula. 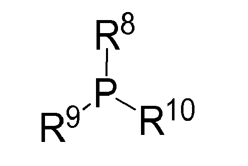 wherein R⁸, R⁹ and R¹⁰ represent a substituted or unsubstituted aryl group, a substituted or unsubstituted alkylaryl group, a substituted or unsubstituted arylalkyl group, or a substituted or unsubstituted linear, branched or cyclic alkyl group.

The phosphine compound used in the second catalyst composition is not particularly limited, but, from a viewpoint of availability and economy, preferable examples thereof are trimethylphosphine, tri-t-butylphosphine, tricyclohexylphosphine, triphenylphosphine, tri(p-tolyl)phosphine, bisdiphenylphosphinoethane and bisdiphenylphosphinopropane.
The relative amounts of the nickel metal source, the fluorine source and the phosphine compound in the second catalyst composition are appropriately chosen in the following ranges: fluorine atom 1 to 10 moles, preferably 1 to 5 moles, per 1 mole of the nickel metal source, and phosphine compound 1 to 10 moles, preferably 1 to 5 moles, per 1 mole of the nickel metal source.

The second catalyst composition can be prepared by combining the above-mentioned nickel metal source, fluorine source and phosphine compound. The procedure of combining the three ingredients includes, for example, a procedure of mixing together the above-mentioned three ingredients, and a procedure of adding the three ingredients separately in a solution for a cross-coupling reaction thereby forming the second catalyst composition within the reactive solution. The state of the three ingredients is not particularly limited, and they may be present discretely, or at least part of the three ingredients may form a complex.

The second catalyst composition may further comprise an organic phosphorus compound. The organic phosphorus compound includes, for example, phosphites, and, as specific examples of the phosphites, there can be mentioned trimethoxyphosphite, triethoxyphosphite and triphenoxyphosphite.
The relative amounts of the phosphine compound and the organic phosphorus compound are such that the amount of the phosphine compound is usually in the range of 1 to 5 moles, preferably 2 to 3 moles, per 1 mole of the organic phosphorus compound.

The second catalyst composition may further comprise, as optional ingredients, an amine compound such as pyridine, triethylamine or N,N,N',N'-tetramethylethylenediamine; a typical metal halide such as zinc chloride, zinc bromide or sodium iodide; and unsaturated hydrocarbons such as ethylene, styrene, butadiene, cyclooctadiene, norbornadiene or diphenylacetylene.
In the case when the second catalyst composition comprises the above-mentioned optional ingredients, the amounts of the optional ingredients are appropriately chosen in the range of 1 to 99% by mole based on the total catalyst composition.

The above-mentioned two types of the catalyst composition, i.e., the first and second catalyst compositions may additionally comprise as other catalyst ingredients at least one metal source selected from the group consisting of an iron metal source and a cobalt metal source.
The iron metal source is capable of giving an iron metal to a catalyst system. The iron metal source is not particularly limited, and, as specific examples thereof, there can be mentioned an iron powder; iron-containing compounds such as iron (II) chloride, iron(III) chloride, iron(II) bromide, iron(III) bromide, iron(II) iodide, iron(II) fluoride, iron(III) fluoride, iron(II) acetate, iron(II) oxalate, iron(III) oxalate, iron(III) citrate, iron(III) perchlorate, iron(III) acetylacetonate, iron(III) nitrate, iron(III) phosphate and iron(II) sulfate; hydrates of these iron-containing compounds; and complex catalysts derived from these iron-containing compounds.

The cobalt metal source is capable of giving a cobalt metal to a catalyst system. The cobalt metal source is not particularly limited, and, as specific examples thereof, there can be mentioned a cobalt powder; cobalt-containing compounds such as cobalt(II) chloride, cobalt(II) bromide, cobalt(II) iodide, cobalt(II) fluoride, cobalt(II) acetate, cobalt(III) acetate, cobalt(II) formate, cobalt(II) oxalate, cobalt(II) benzoate, cobalt(II) stearate, cobalt(II) borate, cobalt(II) acetylacetonate, cobalt(III) acetylacetonate, cobalt(II) carbonate, cobalt(II) sulfate, cobalt (II) nitrate and cobalt (II) phosphate; hydrates of these cobalt-containing compounds; and complex catalysts derived from these cobalt-containing compounds.
The relative amounts of the iron metal source and the cobalt metal source in each of the first and second catalyst compositions are such that the amount of each of the iron metal source and the cobalt metal source in each catalyst composition is in the range of 0.1 to 5 moles per 1 mole of the nickel metal source.

### [Process for Producing Cross-Coupled Compound]

In accordance with the present invention, there is further provided a process for producing a cross-coupled compound represented by the following general formula (5):

**R⁵-R⁶** (5)

wherein R⁵ and R⁶ represent a substituted or unsubstituted aryl group, a substituted or unsubstituted heteroaryl group, a substituted or unsubstituted linear, branched or cyclic alkyl group, or a substituted or unsubstituted linear, branched or cyclic alkenyl group,
which comprises allowing an organometallic compound represented by the following general formula (2) or (3):

**R⁵ - MY¹** (2)

**R⁵ - M - R⁵** (3)

wherein R⁵ is the same as defined above, M represents a magnesium metal or a zinc metal, and Y¹ represents a halogen atom,
to react with a compound represented by the following general formula (4):

**R⁶ - Y²** (4)

wherein R⁶ is the same as defined above, and Y² represents a halogen atom, a methanesulfonate group, a toluenesulfonate group or a trifluoromethanesulfonate group,
in the presence of a catalyst composition selected from the above-mentioned first catalyst composition and the second catalyst composition.

In the above formulae (2) to (5), a substituted or unsubstituted aryl group for R⁵ and R⁶ includes, for example, substituted or unsubstituted aryl groups having 1 to 4 rings and 5 to 18 carbon atoms. As specific examples of the aryl groups, there can be mentioned phenyl, naphthyl, anthracenyl, azulenyl, pyrenyl, phenanthrenyl and fluorenyl groups. Optional number of hydrogen atoms in these aryl groups may be substituted by substituents which include, for example, a halogen atom, a substituted or unsubstituted liner, branched or cyclic alkyl group, a substituted or unsubstituted aryl group, a substituted or unsubstituted heteroaryl group, a substituted or unsubstituted liner, branched or cyclic alkenyl group, a hydroxyl group, an alkoxy group, an amino group, a cyano group, a carbonyl group, a carboxyl group and an ester group.

A substituted or unsubstituted heteroaryl group for R⁵ and R⁶ includes, for example, substituted or unsubstituted heteroaryl groups having 1 to 4 rings and 5 to 18 carbon atoms. As specific examples of the heteroaryl groups, there can be mentioned pyridyl, pyrimidyl, pyridazyl, pyrazyl, triazyl, benzofuranyl, indolyl, benzothiofenyl, quinolyl, isoquinolyl, carbazolyl, acridinyl, phenanthrolinyl and phenothiazinyl groups.
Optional number of hydrogen atoms in these heteroaryl groups may be substituted by substituents which include, for example, a halogen atom, a substituted or unsubstituted liner, branched or cyclic alkyl group, a substituted or unsubstituted aryl group, a substituted or unsubstituted heteroaryl group, a substituted or unsubstituted liner, branched or cyclic alkenyl group, a hydroxyl group, an alkoxy group, an amino group, a cyano group, a carbonyl group, a carboxyl group and an ester group.

A substituted or unsubstituted linear, branched or cyclic alkyl group for R⁵ and R⁶ includes, for example, linear, branched or cyclic alkyl groups having 1 to 20 carbon atoms. As specific examples of the alkyl groups, there can be mentioned methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, eicosyl, cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentadecyl, cyclohexadecyl, cycloheptadecyl, cyclooctadecyl, cyclononadecyl and cycloeicosyl groups. Further, the substituted or unsubstituted alkyl group includes polycyclic hydrocarbon group such as a norbornal group and a adamantyl group.
Optional number of hydrogen atoms in these alkyl groups may be substituted by substituents which include, for example, a halogen atom, a substituted or unsubstituted liner, branched or cyclic alkyl group, a substituted or unsubstituted aryl group, a substituted or unsubstituted heteroaryl group, a substituted or unsubstituted liner, branched or cyclic alkenyl group, a hydroxyl group, an alkoxy group, an amino group, a cyano group, a carbonyl group, a carboxyl group and an ester group.

A substituted or unsubstituted liner, branched or cyclic alkenyl group for R⁵ and R⁶ includes, for example, alkenyl groups having 2 to 20 carbon atoms. As specific examples of the alkenyl groups, there can be mentioned vinyl, propenyl, butenyl, pentenyl, hexenyl, heptenyl, octenyl, nonenyl, decenyl, undecenyl, dodecenyl, tridecenyl, tetradecenyl, pentadecenyl, hexadecenyl, heptadecenyl, octadecenyl, nonadecenyl, eicosenyl, cyclopropenyl, cyclobutenyl, cyclopentenyl, cyclohexenyl, cycloheptenyl, cyclooctenyl, cyclononenyl, cyclodecenyl, cycloundecenyl, cyclododecenyl, cyclotridecenyl, cyclotetradecenyl, cyclopentadecenyl, cyclohexadecenyl, cycloheptadecenyl, cyclooctadecenyl, cyclononadecenyl and cycloeicosenyl groups. Further, the substituted or unsubstituted alkenyl group includes polycyclic hydrocarbon group such as a norbornyl group.
Optional number of hydrogen atoms in these alkenyl groups may be substituted by substituents which include, for example, a halogen atom, a substituted or unsubstituted liner, branched or cyclic alkyl group, a substituted or unsubstituted aryl group, a substituted or unsubstituted heteroaryl group, a substituted or unsubstituted liner, branched or cyclic alkenyl group, a hydroxyl group, an alkoxy group, an amino group, a cyano group, a carbonyl group, a carboxyl group and an ester group.

A halogen atom for Y¹ includes, for example, a fluorine atom, a chlorine atom, a bromine atom and an iodine atom.
The amount of the catalyst composition used for the cross-coupling according to the present invention is such that the amount of nickel atom in the catalyst composition is in the range of 0.001 to 0.15 equivalent, preferably 0.001 to 0.05 equivalent, based on the compound represented by the formula (4). When the amount is smaller than 0.001 equivalent, the reaction does not proceed smoothly. In contrast, when the amount is larger than 0.15 equivalent, the yield does not increase with an increase of the catalyst amount and the process is disadvantageous from cost consideration.

In the process for producing the cross-coupled compound according to the present invention, the relative amount of the compound represented by the formula (2) or (3) and the compound represented by the formula (4) is such that the compound of formula (4) is in the range of 0.5 to 1 mole, preferably 0.7 to 1 mole, per 1 mole of the compound of formula (2) or (3).
The procedures adopted in the process for cross-coupling according to the present invention vary depending upon the reaction medium and other conditions, but the cross-coupling reaction is carried usually at a temperature in the range of 0 to 150°C, preferably 60 to 120°C, usually for 6 to 48 hours, preferably 12 to 36 hours, in an argon or nitrogen gas atmosphere.

The catalyst composition in the process of the present invention may further comprise, as optional ingredients, an amine compound such as pyridine, triethylamine or N,N,N',N'-tetramethylethylenediamine; a typical metal halide such as zinc chloride, zinc bromide or sodium iodide; and unsaturated hydrocarbons such as ethylene, styrene, butadiene, cyclooctadiene, norbornadiene or diphenylacetylene.
In the case when the catalyst composition used comprises the above-mentioned optional ingredients, the amounts of the optional ingredients are appropriately chosen in the range of 1 to 10 moles per 1 mole of the compound of the formula (4).

The cross-coupling reaction is usually carried out using the conventional reaction medium which does not give a baneful influence on the reaction. As specific examples of the reaction medium, there can be mentioned ether solvents such as tetrahydrofuran (THF), diethyl ether, tetrahydropyran (THP), 1,4-dioxane, dibutyl ether, methylcyclohexyl ether and 1,2-dimethoxyethane; aprotic solvents such as N,N-dimethylformamide, N,N-dimethylacetamide, dimethylsulfoxide, N-methylpyrrolidone (NMP) and hexamethylphosphoramide (HMPA); halogenated hydrocarbon solvents such as methylene chloride, ethylene chloride and 1,2-dichlorobenzene; aromatic hydrocarbon solvents such as benzene, toluene, xylene and mesitylene; and other organic solvents.
After completion of the cross-coupling reaction, washing such as acid washing, water washing and/or aqueous alkali washing are carried out to remove inorganic by-products and unreacted raw materials from a reaction product. Ordinary purification technique such as chromatography, distillation and recrystallization can be adopted to give a target cross-coupled compound.

### [Examples]

The present invention will now be specifically described by the following examples that by no means limits the scope of the invention.

### (Conditions for Gas Chromatography (GC) Analysis)

GC analysis equipment: GC-17A available from Shimadzu Corporation
GC column: HR-1 (length 25 m, inner diameter 0.25 mm, membrane thickness 0.25 µm, available from Shinwa Chem. Ind., Ltd. :
Column temperature: maintained at 40°C for 3 minutes and then elevated to 200°C at a rate of 10°C/min.
Injection area temperature: 300°C
Detection area temperature: 300°C (FID)

### Example 1

### [Synthesis of 4-methylbiphenyl]

Chlorobenzene (327.5 mg, 2.9 mmol, available form Wako Pure Chem. Ind., Ltd.) and a solution (3.18 mL) in tetrahydrofuran (THF) of p-tolylmagnesium bromide (1.13 M, 3. 6 mmol, available from Tokyo Chem. Ind., Ltd.) were added to a mixture of NiF₂ (2.90 mg, 0.03 mmol, available from Sigma-Aldrich Corp.) and 1,3-bis(2,6-diisopropylphenyl)imidazolium chloride (25.8 mg, 0.06 mmol, available from Sigma-Aldrich Corp.) at 0°C in an argon gas atmosphere, and the obtained mixture was reacted at 60°C for 36 hours. Then, the reaction mixture was cooled to room temperature, and 2.0 ml of a saturated aqueous ammonium chloride solution was added to the reaction mixture. Further, 3.0 ml of hexane was added thereto and an organic phase was separated. Extraction of an aqueous phase with diethyl ether was conducted five times. Gas chromatography analysis of the collected organic extracts revealed that the yield of target compound was 96%. The amounts of homo-coupled compounds derived from chlorobenzene and p-tolylmagnesium bromide were 0% and 3%, respectively.

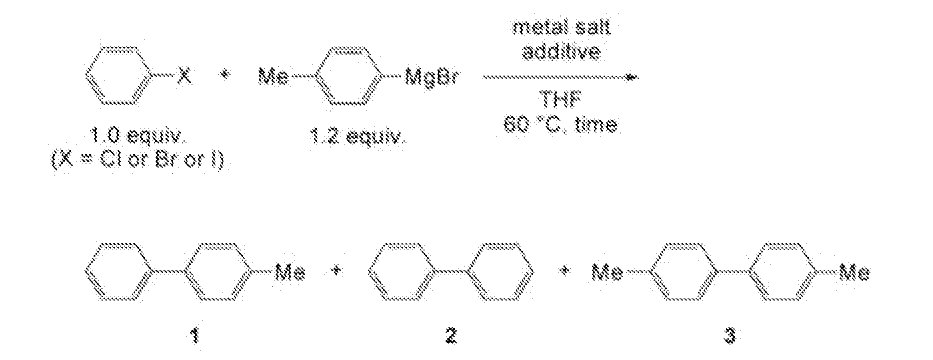

### Examples 2-15

### [Synthesis of 4-methylbiphenyl]

By the same procedures as described in Example 1, the target compound was synthesized wherein the catalyst composition and the organic halide and the reaction conditions, shown in Table 1, were adopted instead of those as adopted in Example 1. All other conditions remained the same. The results obtained in Examples 1-15 are shown in Table 1.

In Table 1, abbreviation "acac" refers to an acetylacetonato group, and the chemical formulae: SIPr•HCl, IPr•HCl, IPr•HBF4, IPr, I-t-Bu•HCl, PPh₃, P(t-Bu)₃ and NiCl₂(dppp) refer to the following compounds, respectively.

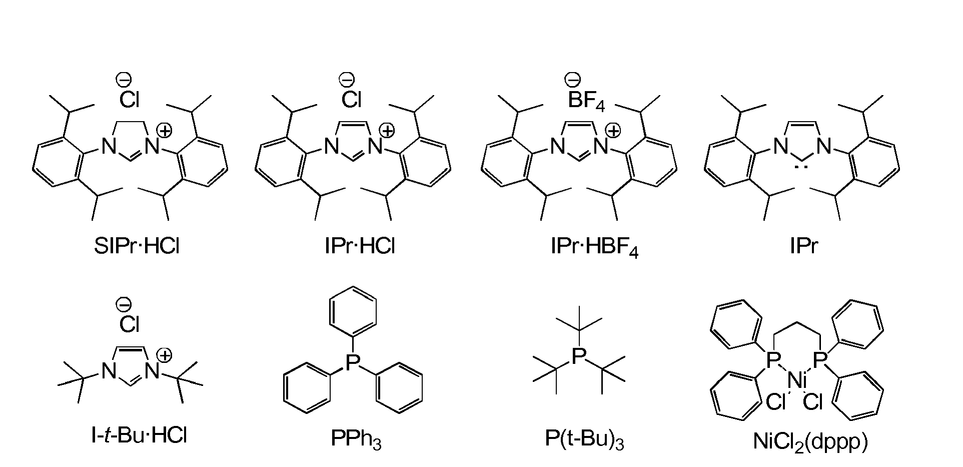

### Comparative Examples 1-5

### [Synthesis of 4-methylbiphenyl]

By the same procedures as described in Example 1, the target compound was synthesized wherein the catalyst composition and the organic halide and the reaction conditions, shown in Table 1, were adopted instead of those as adopted in Example 1. All other conditions remained the same. The results obtained in Comparative Examples 1-5 are also shown in Table 1.

**Table 1**

| | metal salt (mol%) | addive (mol%) | X | time (h) | yield(%) | | |
|---|---|---|---|---|---|---|---|
| | | | | | 1 | 2 | 3 |
| Example 1 | NiF₂(1.0) | IPr·HCl(2.0) | Cl | 18 | 96 | 0 | 3 |
| Example 2 | NiF₂·4H₂O(1.0) | IPr·HCl(2.0) | Cl | 18 | 96 | 0 | 3 |
| Example 3 | NiF_{2·}4H₂O(0.5) | SIPr·HCl(1.5) | Br | 4 | 98 | 1 | 1 |
| Example 4 | NF₂(0.5) | IPr·HCl(0.5) | Br | 4 | 98 | 2 | 4 |
| Example 5 | NiCl₂(0.5), KF(2.0) | IPr·HCl(2.0) | Br | 1 | 89 | 6 | 12 |
| Example 6 | NiF₂(0.5), FeF_{3·}3H₂O(0.5) | IPr·HCl(2.0) | Cl | 8 | 90 | 1 | 4 |
| Example 7 | NiF₂(0.5), CoF₂·4H₂O(0.5) | IPr·HCl(2.0) | Cl | 96 | 94 | 3 | 9 |
| Example 8 | NiF₂(0.33), FeF₃·3H₂O(0.33), CoF₂·4H₂O(0.33) | IPr·HCl(2.0) | Cl | 96 | 84 | 1 | 5 |
| Example 9 | NiF₂(0.5) | IPr-HBF₄(1.0) | Cl | 48 | 98 | 0 | 1 |
| Example 10 | NiF₂(0.5) | I-t-BuHCl(1.0) | Cl | 48 | 96 | 0 | 3 |
| Example 11 | iF₂(0.5) | SIPr·HCl(1.0) | Cl | 48 | 94 | 2 | 10 |
| Example 12 | NiF₂(0.5) | P(tBu)₃(1.0) | Cl | 16 | 98 | 1 | 1 |
| Example 13 | NiF₂(0.5) | IPr(1.0) | Br | 1 | 99 | 0 | 1 |
| Example 14 | NiF₂(1.0) | PPh₃(4.0) | Br | 16 | 98 | 6 | 14 |
| Example 15 | NiF₂(1.0) | IPr-HCl(1.0) | I | 1 | 92 | 1 | 11 |
| Co.Ex. 1 | NiCl₂(1.0) | IPr·HCl(2.0) | Cl | 15 | 64 | 18 | 23 |
| Co.Ex. 2 | Ni(acac)₂(5.0) | IPr·HCl(5.0) | Cl | 4 | 46 | 11 | 30 |
| Co.Ex. 3 | NiF₂(0.9) | -- | Br | 22 | 59 | 32 | 40 |
| Co.Ex. 4 | NiCl₂(dppp)(0.5) | -- | Br | 8 | 80 | 3 | 25 |
| Co.Ex. 5 | NiCl₂(1.0) | PPh₃(4.0) | Br | 1 | 84 | 8 | 22 |

### Example 16

### [Synthesis of 4'-methoxy-3,5-difluorobiphenyl]

1-Bromo-3,5-difluorobenzene (589.0mg, 3.05mmol, available form Tokyo Chem. Ind., Ltd.) and a THF solution (4.10 mL) of p-methoxyphenylmagnesium bromide (0.88 M, 3.6 mmol, available from Tokyo Chem. Ind., Ltd.) were added to a mixture of NiF₂ (1.45 mg, 0.015 mmol, available from Sigma-Aldrich Corp.) and 1,3-bis(2,6-diisopropylphenyl)imidazolium chloride (12.8 mg, 0.03 mmol, available from Sigma-Aldrich Corp.) at 0°C in an argon gas atmosphere, and the obtained mixture was reacted at 60°C for 3 hours. Then, the reaction mixture was cooled to room temperature, and 2.0 ml of a saturated aqueous ammonium chloride solution was added to the reaction mixture. Further, 3.0 ml of hexane was added thereto, and an organic phase was separated. Extraction of an aqueous phase with diethyl ether was conducted five times. Gas chromatography analysis of the collected organic extracts revealed that the yield of target compound was 80%. The amounts of homo-coupled compounds derived from 1-bromo-3,5-difluorobenzene and p-methoxyphenylmagnesium bromide were 0% and 4%, respectively.

### Examples 17-26

By the same procedures as in Example 16, cross-coupled compounds (Ar¹-Ar²) were synthesized wherein the same catalyst composition as used in Example 16 was used, and the organic halide (Ar¹-X) and the organomagnesium compound (Ar²MgBr) and the reaction conditions, which are shown in Table 2, were adopted instead of those as adopted in Example 16. All other conditions remained the same. The results obtained in Examples 16-26 are shown in Table 2.

**Ar¹-X + Ar²MgBr → Ar¹-Ar²**

**Table 2**

| | Ar¹-X | Ar²MgBr (equiv) | NiF₂ (X mol%) IPr·HCl (2X mol%) | conditions | yield (%) |
|---|---|---|---|---|---|
| Example 16 | 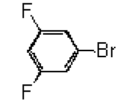 | 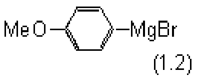 | X = 0.5 | 60 °C, 3 h | **80** |
| Example 17 | 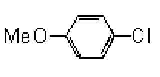 | 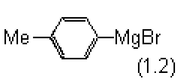 | X = 3 | 60 °C, 24 h | **88** |
| Example 18 | 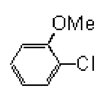 | 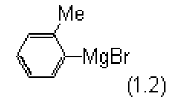 | X = 1 | 60 °C, 36 h | **94** |
| Example 19 | 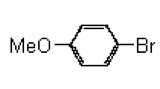 | 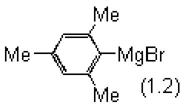 | X = 1 | 80 °C, 12 h | **98** |
| Example 20 | 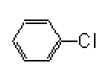 | 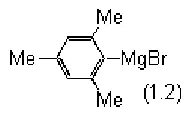 | X = 1 | 120 °C, 4 h (in toluene) | **96** |
| Example 21 | 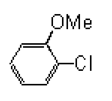 | 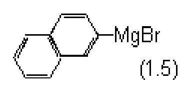 | X = 3 | 60 °C, 24 h | **82** |
| Example 22 | 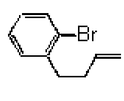 | 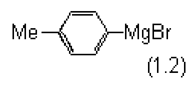 | X = 1 | 60 °C, 18 h | **94** |
| Example 23 | 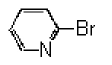 | 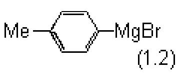 | X = 0.5 | 60 or, 16 h | **93** |
| Example 24 | 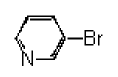 | 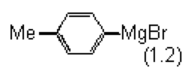 | X = 0.5 | 60 °C, 16 h | **84** |
| Example 25 | 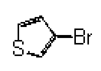 | 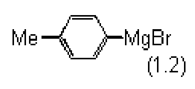 | X = 0.5 | 60 °C, 18 h | **83** |
| Example 26 | 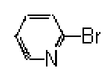 | 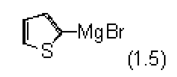 | X = 3 | 80 °C, 24 h | **99** |

### Example 27

### [Synthesis of 4-methylbiphenyl]

A THF solution (3.13 mL) of p-tolylmagnesium bromide (0.96 M, 3.0 mmol, available from Tokyo Chem. Ind., Ltd.) was added to a zinc chloride·N,N,N',N'-tetramethylethylenediamine complex (378.8 mg, 1.5 mmol, available from Sigma-Aldrich Corp.) at 0°C in an argon gas atmosphere. To the obtained mixture, NiF₂ (2.90 mg, 0.03 mmol, available from Sigma-Aldrich Corp.), 1,3-bis(2,6-diisopropylphenyl)imidazolium chloride (25.5 mg, 0.06 mmol, available from Sigma-Aldrich Corp.) and bromobenzene (157.0 mg, 1.0 mmol, available form Wako Pure Chem. Ind., Ltd.) were added, and the obtained mixture was reacted at room temperature for 24 hours. Then, 3.0 ml of a saturated aqueous ammonium chloride solution was added to the reaction mixture, and then 3.0 ml of hexane was added thereto. The reaction mixture was filtered by using a celite pad. The celite pad was washed with diethyl ether, and the organic phase was separated from the aqueous phase and then dried over sodium sulfate anhydride. Gas chromatography analysis of the dried organic phase revealed that the yield of target compound was 98%. The amounts of homo-coupled compounds derived from bromobenzene and p-tolylmagnesium bromide were 0% and 1%, respectively.

### Examples 28-30

By the same procedures as in Example 27, cross-coupled compounds (Ar¹-Ar²) were synthesized wherein the same catalyst composition as used in Example 27 was used, and the organic halide (Ar¹-X) and the organozinc compound (Ar²)₂Zn and the reaction conditions, which are shown in Table 3, were adopted instead of those as adopted in Example 27. All other conditions remained the same. The results obtained in Examples 27-30 are shown in Table 3.

**Ar¹ - X + (Ar²)₂Zn → Ar¹ - Ar²**

| | Ar¹-X | (Ar²)₂Zn (equiv) | NiF₂ (X mol%) IPrHCl (2X mol%) | conditions | yield (%) |
|---|---|---|---|---|---|
| Example 27 | 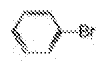 | 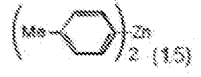 | X = 3 | it, 24 h | **98** |
| Example 28 | 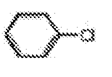 | 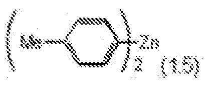 | X = 3 | 60 °C 24 h | **99** |
| Example 29 | 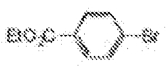 | 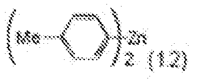 | X = 3 | 60 °C 24 h | **89** |
| Example 30 | 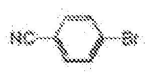 | 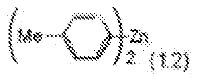 | X = 3 | 60 °C 24 h | **80** |

### Industrial Applicability

The catalyst composition according to the present invention is expected to be applied not only for the coupling reaction using a Grignard compound as in the production process of the present invention, but also for other coupling reactions, such as, for example, a coupling reaction using an organoboron compound, a coupling reaction using an organozinc compound, a coupling reaction using an organosilicon compound, a coupling reaction using an organotin compound, a Heck reaction and a Heck-amination reaction.
Typical examples of the cross-coupled compounds produced by the process using the catalyst composition according to the present invention are unsymmetrical biaryl compounds suitable as liquid crystals and intermediates for medicines.

## Claims

1. A catalyst composition for a cross-coupling reaction comprising a nickel metal source, a fluorine source, and a nitrogen-containing heterocyclic compound represented by the following formula (1A) or (1B), or a phosphine compound: 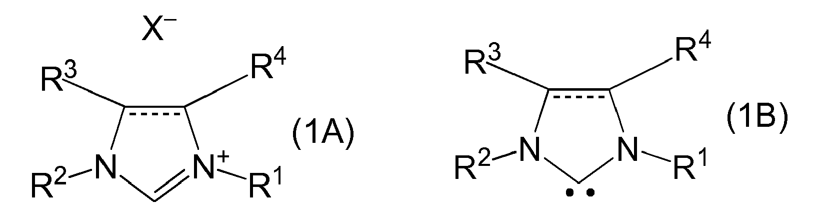 wherein,
R¹ and R² may be the same or different and represent a substituted or unsubstituted aryl group, a substituted or unsubstituted heteroaryl group, a substituted or unsubstituted linear, branched or cyclic alkyl group, or a substituted or unsubstituted linear, branched or cyclic alkenyl group,
R³ and R⁴ may be the same or different and represent a hydrogen atom, a substituted or unsubstituted aryl group, a substituted or unsubstituted heteroaryl group, a substituted or unsubstituted linear, branched or cyclic alkyl group, a substituted or unsubstituted linear, branched or cyclic alkenyl group, a substituted or unsubstituted alkoxy group, a substituted or unsubstituted aryloxy group, or a substituted or unsubstituted silyl group, and R³ and R⁴ may be bonded together to form a ring with the carbon atoms to which R³ and R⁴ are bonded, and
-̅-̅-̅-̅-̅-̅
represents a single bond or a double bond, and
X⁻ represents a univalent anion.

2. The catalyst composition for a cross-coupling reaction according to claim 1, wherein the phosphine compound is tri-t-butylphosphine or triphenylphosphine.

3. The catalyst composition for a cross-coupling reaction according to claim 1 or 2, wherein the nickel metal source and the fluorine source are nickel fluoride.

4. The catalyst composition for a cross-coupling reaction according to any one of claims 1 to 3, which further comprises at least one compound selected from the group consisting of an organic phosphorus compound, an amine compound, a typical metal halide and an unsaturated hydrocarbon.

5. The catalyst composition for a cross-coupling reaction according to any one of claims 1 to 4, which further comprises at least one metal source selected from the group consisting of an iron metal source and a cobalt metal source.

6. A process for producing a cross-coupled compound represented by the following general formula (5):
**R⁵-R⁶** (5)
wherein R⁵ and R⁶ represent a substituted or unsubstituted aryl group, a substituted or unsubstituted heteroaryl group, a substituted or unsubstituted linear, branched or cyclic alkyl group, or a substituted or unsubstituted linear, branched or cyclic alkenyl group,
which comprises allowing an organometallic compound represented by the following general formula (2) or (3):
**R⁵ - MY¹** (2)
**R⁵ - M - R⁵** (3)
wherein R⁵ is the same as defined above, M represents a magnesium metal or a zinc metal, and Y¹ represents a halogen atom,
to react with a compound represented by the following general formula (4):
**R⁶ - Y²** (4)
wherein R⁶ is the same as defined above, and Y² represents
a halogen atom, a methanesulfonate group, a toluenesulfonate group or a trifluoromethanesulfonate group,
in the presence of the catalyst composition as claimed in any one of claims 1 to 5.

7. A process for producing a cross-coupled compound represented by the following general formula (5):
**R⁵-R⁶** (5)
wherein R⁵ and R⁶ represent a substituted or unsubstituted aryl group, a substituted or unsubstituted heteroaryl group, a substituted or unsubstituted linear, branched or cyclic alkyl group, or a substituted or unsubstituted linear, branched or cyclic alkenyl group,
which comprises allowing an organomagnesium compound represented by the following general formula (6):
**R⁵ - MgY¹** (6)
wherein R⁵ is the same as defined above, and Y¹ represents a halogen atom,
to react with a compound represented by the following general formula (4):
**R⁶ - Y²** (4)
wherein R⁶ is the same as defined above, and Y² represents a halogen atom, a methanesulfonate group, a toluenesulfonate group or a trifluoromethanesulfonate group,
in the presence of the catalyst composition as claimed in any one of claims 1 to 5.
